# EUROPÄISCHE PATENTSCHRIFT

(11) **EP 1 682 058 B1**
(45) Veröffentlichungstag und Bekanntmachung des Hinweises auf die Patenterteilung: **22.08.2007**
(21) Anmeldenummer: 04790610.2
(22) Anmeldetag: 18.10.2004
(51) Int. Cl.: A61F 9/01, A61F 9/009

(54) **ADAPTER ZUM KOPPELN EINER LASERBEARBEITUNGSVORRICHTUNG MIT EINEM OBJEKT**
ADAPTER FOR COUPLING A LASER PROCESSING DEVICE TO AN OBJECT
ADAPTATEUR PERMETTANT DE COUPLER UN DISPOSITIF D'USINAGE AU LASER ET D'UN OBJET

(30) Priorität: 14.11.2003 DE 10353264
(43) Veröffentlichungstag der Anmeldung: 26.07.2006
(73) Patentinhaber: Carl Zeiss Meditec AG, 07745 Jena (DE)
(72) Erfinder: MÜHLHOFF, Dirk, 07751 Kunitz (DE); EBERT, Elke, 07743 Jena (DE); FESTAG, Karsten, 07749 Jena (DE); WOLF, Uwe, 99441 Magdala (DE)
(74) Vertreter: Geyer, Fehners & Partner
(86) Internationale Anmeldenummer: PCT/EP2004/011785
(87) Internationale Veröffentlichungsnummer: WO 2005/048896

(56) Entgegenhaltungen:
- US-A- 4 558 698
- US-A- 5 548 352
- US-A- 5 549 632

## Beschreibung

Die Erfindung bezieht sich auf einen Adapter zum Koppeln einer Laserbearbeitungsvorrichtung mit einem Objekt, der aufweist einen zentralen Bereich, der in den Strahlengang der Laserbearbeitungsvorrichtung schaltbar ist, einen Beleuchtungsstrahlengang, durch den Beleuchtungsstrahlung zur Beleuchtung eines durch den zentralen Bereich erfaßbaren Objektfeldes leitbar ist, und einen außerhalb des zentralen Bereiches liegenden Randbereich, mit dem der Adapter am Objekt und/oder an der Laserbearbeitungsvorrichtung befestigbar ist.

Bei der Materialbearbeitung mittels Laserstrahlung wird oft eine Laserbearbeitungsvorrichtung zum Abrastern der zu bearbeitenden Gebiete des Objektes mit dem Laserstrahl eingesetzt. Die Genauigkeit der Positionierung des Laserstrahls bestimmt dabei in der Regel die bei der Bearbeitung erzielte Präzision. Wird der Laserstrahl in ein Bearbeitungsvolumen fokussiert, bedarf es einer exakten dreidimensionalen Positionierung. Für eine hochgenaue Bearbeitung ist es deshalb in der Regel unerläßlich, das Objekt in exakt definierter Lage zur Laserbearbeitungsvorrichtung zu halten. Für solche Anwendungen dient der eingangs genannte Adapter, da mit ihm das zu bearbeitende Objekt fixiert werden kann, wodurch definierte Verhältnisse bis zum Bearbeitungsvolumen erreichbar sind. Der zentrale Bereich des Adapters wird damit Teil des Strahlenganges.

Aus der US 4558698 ist ein Adapter bekannt, über den der Schlemm-Kanal des Auges mit Laserstrahlung beaufschlagt werden kann. Die zweiteilige Form des unabhängigen Anspruchs basiert sich auf diesem Stand der Technik. Die US 5548352 und die US 5549632 beschreiben Kontaktgläser für die Augenchirurgie.

Dies ist insbesondere bei der Mikrobearbeitung von Materialien notwendig, die nur eine geringe lineare optische Absorption im Spektralbereich der bearbeitenden Laserstrahlung aufweisen. Bei solchen Materialien werden üblicherweise nicht-lineare Wechselwirkungen zwischen Laserstrahlung und Material ausgenutzt, meist in Form eines optischen Durchbruches, der im Fokus des Laserstrahls erzeugt wird. Da die bearbeitende Wirkung dann nur im Laserstrahlfokus stattfindet, ist es unerläßlich, den Fokuspunkt exakt dreidimensional auszurichten. Zusätzlich zu einer zweidimensionalen Ablenkung des Laserstrahls ist somit eine exakte Tiefeneinstellung der Fokuslage im Strahlengang erforderlich. Der eingangs genannte Adapter dient dazu, konstante und auch mit einer gewissen Genauigkeit bekannte optische Verhältnisse im Strahlengang zum Objekt sicherzustellen, indem sein zentraler Bereich Teil des Strahlenganges ist und er Objekt und Laserbearbeitungsvorrichtung koppelt.

Eine typische Anwendung für einen solchen Adapter ist das als Femtosekunden-LASIK bekannte augenoptische Operationsverfahren, bei dem die Laserbearbeitungsvorrichtung einen Laserstrahl auf einen Fokuspunkt in der Größenordnung weniger Mikrometer in die Hornhaut fokussiert. Im Fokus entsteht dann ein Plasma, das eine lokale Trennung des Hornhautgewebes bewirkt. Durch geeignete Aneinanderreihung der auf diese Weise erzeugten lokalen Trennungszonen werden makroskopische Schnitte realisiert und ein bestimmtes Hornhautteilvolumen isoliert. Durch Entnahme des Teilvolumens wird dann eine gewünschte Brechungsänderung der Hornhaut erreicht, so daß eine Fehlsichtigkeitskorrektur möglich ist.

Für dieses LASIK-Verfahren ist aus der US 6.373.571 eine mit Referenzmarken versehene Kontaktlinse bekannt. Diese Kontaktlinse wird mittels einer separaten Meßvorrichtung einjustiert, wodurch ein relativ aufwendiger Aufbau bedingt ist. Ein Beispiel für einen Adapter der genannten Art ist in der EP 1 159 986 A2 beschrieben. Er ähnelt der Kontaktlinse der US 6.373.571, weist aber zusätzlich einen Rand in Form einer Halterung mit Strichmarken auf, die dem Chirurgen eine visuelle Ausrichtung ermöglichen.

Bei der Materialbearbeitung mittels Laserstrahlung ergibt sich oft die Notwendigkeit, die Durchführung der Bearbeitung zu überwachen. Man wünscht, das Bearbeitungsfeld während der Applikation der Laserstrahlung beobachten zu können. Dies gilt insbesondere für das genannte LASIK-Verfahren, bei dem der behandelnde Arzt das Operationsfeld beobachten muß. Die genannte Laserbearbeitungsvorrichtung verfügt deshalb in der Regel über ein optisches System zur Abbildung des Gebietes, in dem die Laserstrahlung zur Anwendung kommt. Das Bild entsteht entweder auf einer Kamera oder in einer Zwischenbildebene, aus der dann über ein Okular eine direkte visuelle Betrachtung möglich ist. Die Beobachtung erfolgt durch den zentralen Bereich des Adapters hindurch, und es ist erforderlich, daß die Laserbearbeitungsvorrichtung das Gebiet, in dem die Laserstrahlung zur Anwendung kommt und das als Objektfeld beobachtet wird, beleuchtet.

Für die zur visuellen Beobachtung nötige Beleuchtung könnte man daran denken, eine Lichtquelle zu verwenden, deren Strahlung durch die Laserbearbeitungsvorrichtung läuft. Da die dort vorgesehene Optik jedoch in der Regel eine Vielzahl von Grenzflächen aufweist, welche alle eine gewisse Restreflektivität haben, wird unvermeidlich ein nicht zu vernachlässigender Teil der Beleuchtungsstrahlung in die Abbildung des Objektfeldes übergekoppelt. Dies macht sich in der Bildebene je nach optischer Anordnung als globale Bildaufhellung oder als helle Flecke an festen Stellen des betrachteten Objektfeldes vor allem in dessen Zentrum bemerkbar. In jedem Fall reduzieren die Reflexe die Bildqualität, mit der das Objektfeld beobachtbar ist.

Ein weiterer denkbarer Ansatz wäre in Form einer Pupillentrennung denkbar. Man müßte dann so vorgehen, daß die zur Beleuchtung verwendete Lichtquelle zunächst in eine Pupillenebene des optischen Systems der Laserbearbeitungsvorrichtung abgebildet wird. Die Abbildung müßte dabei so gestaltet werden, daß nur ein äußerer Ring der Pupillenebene Beleuchtungsstrahlung führt und das Zentrum der Pupillenebene nur zur Abbildung des Objektfeldes benutzt wird. Die Reflektivität von Objekten, die sich nahe der Bildebene befinden, könnte dann nicht die Abbildung des Objektfeldes beeinträchtigen. Die Bedingung, daß sich alle reflektierenden Flächen nahe einer Bildebene befinden, kann jedoch bei einem Adapter der genannten Art regelmäßig nicht eingehalten werden, da dessen zentraler Bereich unvermeidlich im Strahlengang liegt und die Lage des Adapters und mithin der möglicherweise reflektierenden Flächen durch die Art des Objektes vorgegeben ist und somit kaum veränderbar und insbesondere nicht unter optischen Gesichtspunkten optimal wählbar ist. Dabei wäre bei auch bei der geschilderten Pupillentrennung mit störender parasitärer Strahlung zu rechnen.

Der Erfindung liegt deshalb die Aufgabe zugrunde, einen Adapter der eingangs genannten Art so auszubilden, daß eine Beleuchtung des Objektfeldes ohne Beeinträchtigung der Qualität des durch den Beobachtungsstrahlengang erfaßten Objektfeldes auf einfache Weise möglich ist.

Diese Aufgabe wird mit einem Adapter gemäß Anspruch 1 gelöst, bei dem der Beleuchtungsstrahlengang im Randbereich verläuft und in den Randbereich eingekoppelte Beleuchtungsstrahlung direkt und/oder über den zentralen Bereich zum Objektfeld leitet.

Es ist also ein wesentlicher Gedanke der Erfindung, den Beleuchtungsstrahlengang und den Beobachtungsstrahlengang so zu trennen, daß keine unerwünschten Reflexionen auftreten können. Dadurch kann eine hohe Bildqualität erreicht werden. Die Erfindung vermeidet es, das Beleuchtungslicht über dieselben Elemente einzubringen, die für die Beobachtung verwendet werden. Statt dessen wird für die Einkopplung der Beleuchtungsstrahlung am Adapter der Randbereich genutzt, der an der Abbildung des Objektfeldes nicht mitwirkt und dafür auch nicht gedacht ist. Die Beleuchtungsstrahlung wird am Randbereich eingespeist. Rückreflexe in den Beobachtungsstrahlengang der Abbildung sind damit weitgehend verhindert. Dies schließt nicht aus, daß die am Randbereich zugeführte Behandlungsstrahlung auch über den an der Abbildung teilhabenden, zentralen Bereich des Adapters geführt wird. Durch die Zuführung über den Randbereich können Störungen der Abbildung nahezu vollständig vermieden werden.

Der erfindungsgemäße Adapter ermöglicht eine feste Kopplung zwischen der Laserbearbeitungsvorrichtung und dem Objekt und zugleich eine optische Beobachtung des Objektes mit guter Qualität zu erreichen. Der Adapter ist deshalb besonders für chirurgische Verfahren, insbesondere am Auge, geeignet. Zu diesem Zweck weist der zentrale Bereich ein Kontaktglas zum Auflegen auf das Objekt auf und stellt der Randbereich eine Fassung des Kontaktglases der Üblicherweise wird ein solches Kontaktglas zur Durchführung des chirurgischen Verfahrens auf das Auge aufgesetzt und dort z.B. durch Vakuum fixiert. Um eine gute optische Qualität des Kontaktglases sicherzustellen bietet es sich an, die Vakuumfixierung an der Fassung vorzunehmen. Es ist deshalb zweckmäßigerweise an der dem Objekt zugewandten Seite der Fassung eine entsprechende Fixiervorrichtung vorzusehen, die es erlaubt, den Adapter am Objekt zu fixieren. Verwendet man eine Vakuumfixierung, kann die Fassung geeignete Saugkanäle aufweisen, um den Adapter durch Unterdruck auf das Auge zu saugen.

Die optischen Eigenschaften des Kontaktglases werden durch seine Vorderfläche und seine Rückfläche, d.h. die dem Objekt zugewandte Rückfläche und die der Laserbearbeitungsvorrichtung zugewandte Vorderfläche definiert. Beide Flächen können entweder plan sein, oder aber konvex oder konkav gekrümmt oder auch asphärisch ausgebildet werden. Das Kontaktglas muß für die Strahlung der Laserbearbeitungsvorrichtung transparent sein. Dies gilt sowohl für die Beobachtungsstrahlung als auch für eventuell durch die Laserbearbeitungsvorrichtung applizierte Behandlungsstrahlung. Als Materialien kommen deshalb übliche optische Gläser, aber auch transparente Kunststoffe in optischer Qualität, z.B. PMMA, Zeonex, etc., in Frage.

Am Randbereich, z.B. die Fassung, wird die Beleuchtungsstrahlung für das Objektfeld eingeleitet. Zu diesem Zweck weist die Fassung ein zur Beleuchtungsstrahlung transparentes Material, insbesondere PMMA, Polycarbonat, Zeonex öder HW 55 auf.

Die Funktion des Adapters kann darin liegen, der zur Laserbearbeitungsvorrichtung hin orientierten Oberfläche des Objektes eine bestimmte Form zu verleihen bzw. für eine bekannte Grenzfläche zu sorgen. Beim eingangs erwähnten LASIK-Verfahren ist es jedoch darüber hinaus notwendig, den Adapter in vorbestimmter mechanischer Lage mit der Laserbearbeitungsvorrichtung, die dann als Lasertherapiegerät ausgebildet ist, zu koppeln. Dies geschieht ebenfalls über den Randbereich, der, wie erwähnt, beispielsweise als Fassung für ein Kontaktglas ausgebildet werden kann. In dieser Ausführungsform ist die zur Laserbearbeitungsvorrichtung orientierte Eingangsseite des Adapters deshalb vorzugsweise mit geeigneten Mitteln zur festen Verbindung mit dem zum Objekt orientierten Ausgang (z.B. distalen Ende) der Laserbearbeitungsvorrichtung bzw. deren optischem System ausgebildet, so daß eine auf die Laserbearbeitungsvorrichtung bezogene feste Fixierung mittels eines Verschlußmechanismusses möglich ist. Für den Verschlußmechanismus kommt dabei beispielsweise die Ausbildung einer Flanschfläche am Randbereich in Frage.

Um die Beleuchtungsstrahlung zum Objektfeld zu leiten, ist erfindungsgemäß am Randbereich eine spezielle Einkoppeleinrichtung für Beleuchtungsstrahlung vorzusehen. Dabei kann es sich beispielsweise um einen Koppler für einen Lichtwellenleiter, der die Beleuchtungsstrahlung zuführt, handeln.

Um die Beleuchtungsstrahlung in optimaler Weise zum Objektfeld leiten zu können, kann vorteilhafterweise die Einkoppeleinrichtung mit abbildender Wirkung versehen werden, insbesondere durch eine konvexe, konkave, zylindrische oder torische Grenzfläche. Um bestimmte spektrale Gegebenheiten bei der Beleuchtung des Objektfeldes zu realisieren, kann alternativ oder zusätzlich die Einkoppeleinrichtung zum Zwecke der spektralen Filterung oder Reflexreduzierung eine dielektrische Schicht oder absorbierende Filter, wie z.B. Farbgläser, aufweisen.

Die in den Randbereich eingebrachte Beleuchtungsstrahlung kann durch geeignete Lichtquellen erzeugt und mit Lichtleitmitteln zum Objektfeld geleitet werden. Eine einfache Realisierung für solche Lichtleitmittel stellen reflektierende Flächen dar. Es ist deshalb zweckmäßig, am Randbereich mindestens eine die Beleuchtungsstrahlung reflektierende Fläche vorzusehen. Auch kann man daran denken, im Randbereich die Beleuchtungsstrahlung reflektierende Lichtleitkanäle auszubilden. Eine besonders einfache Realisierung stellt eine reflektierende Außenfläche dar, bei der zweckmäßigerweise das Prinzip der totalen inneren Reflexion genutzt werden kann, um am Randbereich eingebrachte Beleuchtungsstrahlung zum Objektfeld zu bringen.

In einer Variante des erfindungsgemäßen Adapters kann eine Fassung aus transparentem Kunststoff verwendet werden, in die ein Kontaktglas, beispielsweise ein plano-konkaves Kontaktglas eingeklebt ist. Die Fassung dient dabei als mechanische Verbindung zwischen Kontaktglas und der als Therapiegerät ausgebildeten Laserbearbeitungsvorrichtung. Das Kontaktglas kann mittels eines optisch transparenten Klebestoffes in die Fassung eingeklebt - sein, wodurch die Grenzfläche zwischen Kontaktglas und Fassung optisch transparent ist. Besonders vorteilhaft ist es, wenn die Brechzahl des Klebestoffes eine Brechzahl aufweist, die zwischen der Brechzahl des Materials der Fassung und der Brechzahl des Materials des Kontaktglases liegt, weil dadurch Reflexionsverluste beim Übergang der Beleuchtungsstrahlung von der Fassung in das Kontaktglas minimal sind. Zur weiteren Reduktion der Verluste können auf den Grenzflächen sowohl der Fassung als auch des Kontaktglases optisch wirksame dielektrische Schichten aufgebracht werden.

Sieht man an der Fassung eine Koppelstelle für einen Lichtwellenleiter vor, kann die Beleuchtungsstrahlung aus einer Lichtquelle, z.B. eines Glühemissionsstrahlers oder einer Leuchtdiode, einfach über einen Lichtwellenleiter eingekoppelt werden. In einer zweckmäßigen Weiterbildung werden Lichtwellenleiterbündel verwendet. Die Einkopplung kann z.B. einen Ellipsoid-Spiegel verwenden, wie es dem Fachmann bekannt ist. Die Lage der Eintrittsstelle kann fast an beliebiger Stelle am Randbereich liegen kann. Durch geeignete Reflexion, beispielsweise durch totale innere Reflexion, wird die Strahlung innerhalb der Fassung geführt, bis sie auf die Grenzfläche vom Kontaktglas trifft. Hier koppelt die Strahlung in das Kontaktglas über. Liegt das Kontaktglas am zu therapierenden Auge an, so koppelt die Strahlung nun in die Cornea über und wird von dort in tiefere Ebenen des Auges geführt. Trifft sie dabei auf die Iris oder auf durch die Lasertherapie hervorgerufene Streuzentren, so wird sie gestreut, und kann über den Beobachtungsstrahlengang der Laserbearbeitungsvorrichtung, d.h. über das Kontaktglas und die Optik der Laserbearbeitungsvorrichtung, abgebildet werden.

In einer Modifikation werden mehrere Einkoppeleinrichtungen verwendet, da sich dann eine gleichmäßige Ausleuchtung des Objektfeldes erzielen läßt. Verwendet man die Variante mit reflektierenden Flächen in Kombination mit mehreren Einkoppeleinrichtungen, kann man die reflektierenden Flächen in Facetten segmentieren und jeder Einkoppeleinrichtung eine Facette oder eine Gruppe von Facetten zuordnen. Zusätzlich erhöht man durch getrennte Ansteuerung der die Einkoppeleinrichtungen speisenden Lichtquellen eine selektive Beleuchtung des Objektfeldes. Zum Beispiel sind Beleuchtungsmodi realisierbar, bei denen das Licht selektiv nur von einer Seite auf das Objektfeld einstrahlt.

Verleiht man den reflektierenden Flächen, oder allgemein der Leitung der Beleuchtungsstrahlung im Randbereich, abbildende Wirkung, kann erreicht werden, daß Licht, welches z.B. nach Verlassen des Lichtleiters sich divergent ausbreitet, gezielt kollimiert wird. Insbesondere ist es möglich, sphärische Oberflächen zu verwenden. Ein schräger Einfall des Lichtes auf diese Fläche kann in einem Astigmatismus resultieren, der gezielt genutzt werden kann, um das Licht so zu formen, daß in einer Schnittebene parallel zur optischen Achse des Beobachtungsstrahlengangs der Divergenzwinkel sehr klein ist, senkrecht dazu aber sehr groß. Damit wird das gesamte Objektfeld in einer Ebene senkrecht zur optischen Achse der Laserbearbeitungsvorrichtung, d.h. deren Beobachtungsstrahlengangs, effektiv ausgeleuchtet.

In einer optionalen Ausgestaltung kann der Randbereich eine separate Austrittsfläche für Beleuchtungsstrahlung aufweisen, die es ermöglicht, auch ein vom Adapter noch beabstandetes Objekt, insbesondere natürlich das menschliche Auge im Falle eines LASIK-Therapiegerätes, zu beleuchten, das noch nicht am zentralen Bereich, z.B. am Kontaktglas, des Adapters anliegt. Dies kann für die Justierung des Objektes auf die optische Achse der Laserbearbeitungsvorrichtung wichtig sein. Diese weitere Austrittsfläche kann natürlich auch eine bestimmte abbildende Wirkung aufweisen, indem man eine bestimmte Oberflächenform oder -eigenschaft wählt. Besonders vorteilhaft sind z.B. sphärische Oberflächenformen, welche eine Abbildung einer Endfläche eines Lichtleiters in eine bestimmte Ebene erlauben. Alternativ kann auch eine torische Form oder die Form eines umlaufenden Wulstes verwendet werden, der sich sehr einfach durch Drehbearbeitung des Materials des Adapters realisieren läßt. Eine solche torische Form führt darüber hinaus vorteilhaft zur Auffächerung der Beleuchtungsstrahlung und damit zu einer gleichmäßigen Beleuchtung des Objektfeldes, auch wenn das Objekt noch nicht am zentralen Bereich des Adapters anliegt.

Die Austrittsfläche, über der die Beleuchtungsstrahlung am Randbereich des Adapters direkt oder über den zentralen Bereich zum Objekt geführt wird; kann alternativ oder teilweise als Streufläche gestaltet sein. Damit erreicht man eine besonders starke Homogenisierung der Beleuchtungsstrahlung am Objektfeld.

Um einen besonders kompakten Aufbau zu erreichen, ist es vorteilhaft, die Einkoppeleinrichtung mit einer am Randbereich befestigten oder in den Randbereich eingelassenen LED auszustatten. Diese Alternative zu einem Lichtleiter führt zu einem sehr kompakten Aufbau. Dabei kann die LED direkt in den Randbereich, z.B. eine Fassung für ein Kontaktglas, eingeklebt oder eingegossen sein. Zur Kontaktierung der LED sind geeignete entsprechende Anschlüsse vorgesehen.

Natürlich ist dieses Konzept nicht auf die Verwendung einer einzelnen LED beschränkt, es können auch mehrere LED verwendet werden, wobei die Möglichkeit gegeben ist, alle LED über ein Kontaktpaar anzusteuern oder jede LED einzeln über eigene elektrische Kontakte anzuschließen. Ebenso ist es möglich, LED zu Gruppen zusammenzufassen und jede Gruppe mit eigenen Kontakten auszustatten.

Besonders vorteilhaft ist es, die Kontakte als ringförmige Kontaktflächen auszulegen. Dies hat den Vorteil, daß der Adapter in beliebiger Drehstellung aufgesetzt werden kann und in jeder Position die LED kontaktiert werden können. Die ringförmige Kontaktfläche muß nicht zwingend am Adapter ausgebildet werden, alternativ ist es auch möglich, geeignete Ringgegenkontakte zu verwenden.

In einer weiteren Ausgestaltung der Kontaktierung der LED erfolgt die Stromzuführung ohne mechanische Kontakte. Statt dessen wird eine induktive oder kapazitive Kopplung eingesetzt. Zu diesem Zweck ist eine geeignete Spule vorgesehen, die beispielsweise an der Laserbearbeitungsvorrichtung befestigt sein kann. Die Spule erzeugt ein elektromagnetisches Wechselfeld, das in einer im Randbereich des Adapters vorgesehenen Spule eine Wechselspannung induziert; mit der die LED betrieben wird. Auf gleicher Weise ist auch eine kapazitive Energieübertragung möglich. Dafür ist es sinnvoll, am Adapter und der Speiseeinrichtung zwei ausgedehnte leitende Flächen auszubilden, welche sich paarweise direkt gegenüberstehen und einen elektrischen Kondensator bilden. Legt man ein Wechselfeld an, erreicht man eine Energieübertragung zur LED.

Als LED kommen Weißlicht-LED oder auch farbige LED in Frage. Bei farbigen LED bietet es sich an, LED in Gruppen zusammenzufassen, z.B. jede Gruppe aus rot, grün und blau emittierenden LED zusammenzusetzen. Sind die roten, grünen und blauen LED einzeln ansteuerbar, so kann über die Ansteuerung die Farbtemperatur der Beleuchtung eingestellt werden. Dadurch ist es möglich, gewisse Strukturen, wie z.B. Blutgefäße, durch Veränderung der Farbtemperatur der Beleuchtungsstrahlung z.B. bei grüner Beleuchtung, besonders hervortreten zu lassen.

Möchte man den Adapter zumindest teilweise aus nicht-transparenten Materialien ausbilden, wird regelmäßig eine mehrteilige Bauweise mit einem nicht-transparenten Fassungsmaterial und einem transparenten Kontaktglasmaterial gewählt werden. Als nicht transparentes Material für den Randbereich kommt z.B. Metall oder Keramik in Frage, weiches gegenüber Kunststoff eine höhere Festigkeit und Elastizität aufweist. In einer solchen Metallfassung sind dann geeignete optische Elemente vorgesehen, die die Beleuchtungsstrahlung in Richtung auf das zu beleuchtende Objektfeld leiten. Dabei kann es sich beispielsweise um eine reflektierende Bohrung handeln, die am Randbereich zugeführte Beobachtungsstrahlung weiterleitet. Die Bohrung kann verspiegelte Innenflächen aufweisen und so die Beobachtungsstrahlen durch Vielfachreflexion führen. Dabei sind in der Bohrung auch Abwinkelungen möglich. Die Bohrung kann entweder an der Grenzfläche von der Fassung zum Kontaktglas enden oder im unteren Bereich der Fassung, so daß das geführte Licht austritt und eine Ebene unterhalb des Kontaktglases bestrahlt. Alternativ oder zusätzlich kann in der Bohrung auch ein abbildendes Element, z.B. eine geeignete Linse, vorgesehen werden.

Zum Erreichen besonders niedriger Herstellkosten ist es möglich und vorteilhaft, Randbereich und zentralen Bereich einstückig in einem Bauteil zu realisieren. Damit kommen für die Herstellung beispielsweise Spritzgußtechniken in Frage.

Die Erfindung wird nachfolgend unter Bezugnahme auf die Zeichnung beispielsweise noch näher erläutert. In den Zeichnungen zeigt:
- Fig. 1: eine schematische Darstellung einer Laserbearbeitungsvorrichtung für ein augenchirurgisches Verfahren,
- Fig. 2: eine schematische Darstellung der Augenhornhaut eines Patienten,
- Fig. 3: eine schematische Darstellung von Beleuchtungs- und Beobachtungsstrahlengang bei einem Adapter für ein augenchirurgisches Verfahren,
- Fig.4: eine schematische Schnittdarstellung eines als Kontaktglas mit Fassung realisierten Adapters für ein augenchirurgisches Verfahren,
- Fig. 5: eine schematische Schnittdarstellung eines einstückigen Adapters für ein chirurgisches Verfahren,
- Fig. 6: eine schematische Schnittdarstellung zur Darstellung des Beleuchtungsstrahlenganges in einem Adapter für ein augenchirurgisches Verfahren,
- Fig. 7: einen Adapter mit gegenüber Fig. 6 abgewandelter Bauweise,
- Fig. 8: einen nochmals abgewandelten Adapter,
- Fig. 9: eine Schnittdarstellung für einen Adapter für ein augenchirurgisches Verfahren, bei dem eine Objektfeldbeleuchtung bereits vor dem Aufsetzen auf das Auge möglich ist, und
- Fig. 10: einen Adapter für ein augenchirurgisches Verfahren mit einer LED als Beleuchtungsquelle.

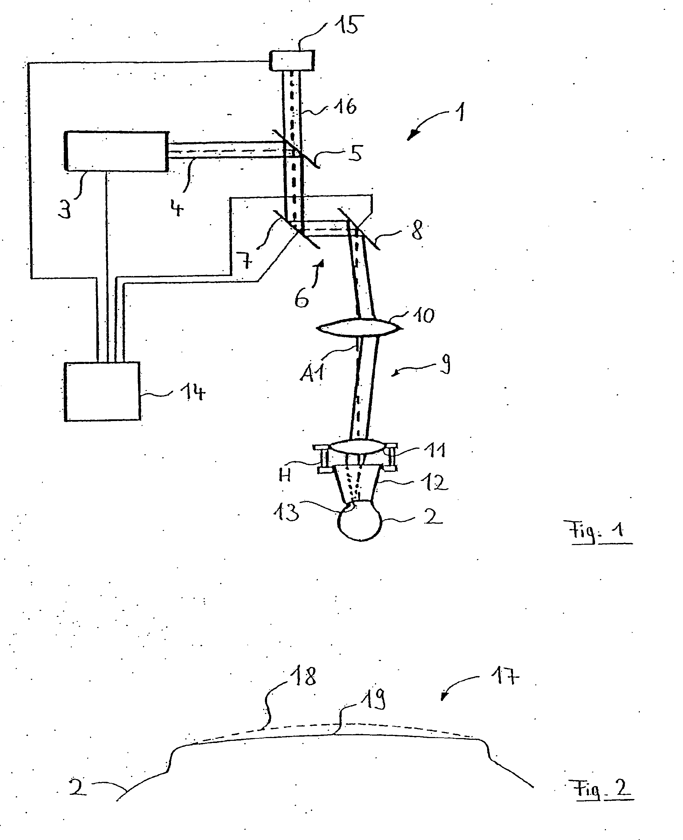

Fig. 1 zeigt ein Behandlungsgerät für ein augenchirurgisches Verfahren ähnlich dem in der EP 1159986 A1 bzw. der US 5549632 beschriebenen. Das Behandlungsgerät 1 der Figur 1 dient dazu, an einem Auge 2 eines Patienten eine Fehlsichtigkeitskorrektur gemäß dem bekannten LASIK-Verfahren auszuführen. Dazu weist das Behandlungsgerät 1 einen Laser 3 auf, der gepulste Laser-Strahlung abgibt. Die Pulsdauer liegt dabei z.B. im Femtosekundenbereich, und die Laserstrahlung wirkt mittels nichtlinearer optischer Effekte in der Hornhaut auf die eingangs beschriebene Art und Weise. Der vom Laser 3 entlang einer optischen Achse A1 abgegebene Behandlungsstrahl 4 fällt dabei auf einen Strahlteiler 5, der den Behandlungsstrahl 4 auf eine Scaneinrichtung 6 leitet. Die Scaneinrichtung 6 weist zwei Scanspiegel 7 und 8 auf, die um zueinander orthogonale Achsen drehbar sind, so daß die Scaneinrichtung 6 den Behandlungsstrahl 4 zweidimensional ablenkt. Eine verstellbare Projektionsoptik 9 fokussiert den Behandlungsstrahl 4 auf bzw. in das Auge 2. Die Projektionsoptik 9 weist dabei zwei Linsen 10 und 11 auf. Das Behandlungsgerät 1 stellt eine Laserbearbeitungsvorrichtung dar.

Der Linse 11 ist ein Adapter 12 nachgeordnet, der über eine Halterung H fest mit der Linse 11 und damit dem Strahlengang des Behandlungsgerätes 1 verbunden ist. Der noch näher zu beschreibende Adapter 12 liegt an der Hornhaut des Auges 2 an. Die optische Kombination aus Behandlungsgerät 1 mit daran befestigtem Adapter 12 bewirkt, daß der Behandlungsstrahl 4 in einem in der Hornhaut des Auges 2 gelegenen Fokus 13 gebündelt wird.

Die Scaneinrichtung 6 wird ebenso wie der Laser 3 und die Projektionsoptik 9 über (nicht näher bezeichnete) Steuerleitungen von einem Steuergerät 14 angesteuert. Das Steuergerät 14 bestimmt dabei die Lage des Fokus 13 sowohl quer zur optischen Achse A1 (durch die Scanspiegel 7 und 8) sowie in Richtung der optischen Achse A1 (durch die Projektionsoptik 9) vor.

Das Steuergerät 14 liest weiter einen Detektor 15 aus, der von der Hornhaut rückgestreute Strahlung, die den Strahlteiler 5 als Rückstrahlung 16 passiert, ausliest. Mittels des Detektors 15 kann der Betrieb des Lasers 3 sehr exakt gesteuert werden.

Der Adapter 12 sorgt dafür, daß die Hornhaut des Auges 2 eine gewünschte Soll-Form erhält. Das Auge 2 befindet sich aufgrund der Anlage der Hornhaut 17 am Adapter 12 in vorbestimmter Lage zum Adapter 12 und damit zum damit verbundenen Behandlungsgerät 1.

Dies ist schematisch in Fig. 2 dargestellt, die einen Schnitt durch die Augenhornhaut 17 zeigt. Um eine exakte Positionierung des Fokus 13 in der Augenhornhaut 17 zu erreichen, muß die Krümmung der Augenhornhaut 17 berücksichtigt werden. Die Augenhornhaut 17 weist eine Ist-Form 18 auf, die von Patient zu Patient unterschiedlich ist. Der Adapter 12 liegt nun an der Augenhornhaut 17 derart an, daß er diese in eine gewünschte Soll-Form 19 verformt. Der genaue Verlauf der Soll-Form 19 hängt dabei von der Krümmung der dem Auge 2 zugewandten Fläche des Adapters 12 ab. Durch den Adapter 12 sind bekannte geometrische und optische Verhältnisse für das Einbringen und Fokussieren des Behandlungsstrahls 4 in die Hornhaut 17 gegeben. Da die Hornhaut 17 am Adapter 12 anliegt und dieser wiederum über die Halterung H gegenüber dem Strahlengang des Behandlungsgerätes 1 ortsfest ist, kann der Fokus 13 durch Ansteuerung der Scaneinrichtung 6 sowie der verstellbaren Projektionsoptik 9 dreidimensional exakt in der Hornhaut 17 positioniert werden.

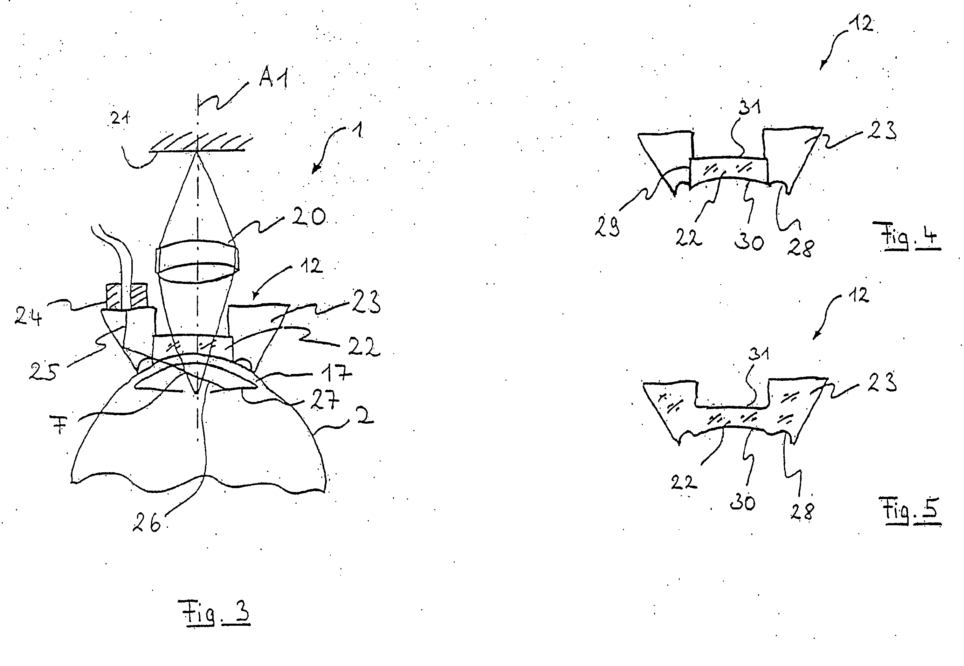

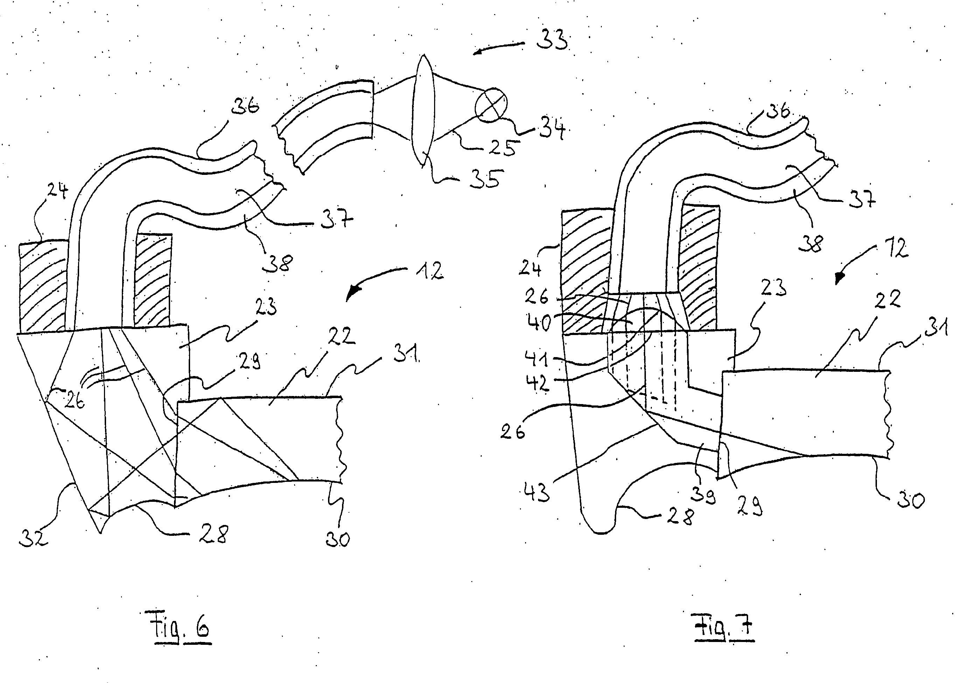

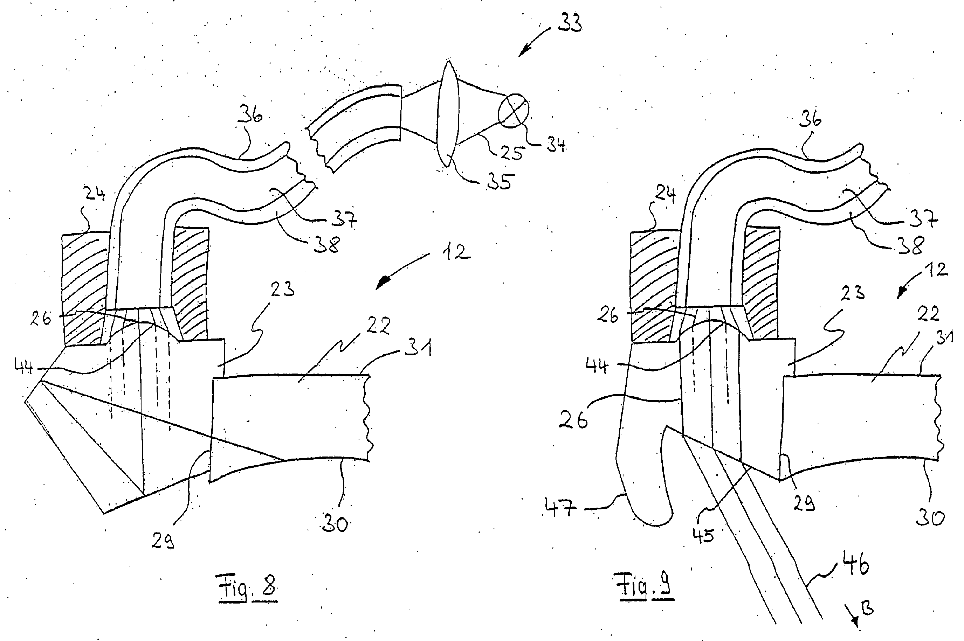

Beim Behandlungsgerät 1 ist es während der Therapie erforderlich, daß der Arzt das Operationsfeld, d.h. die Augenhornhaut 17, beobachtet. Es ist deshalb das in Fig. 3 schematisch dargestellte optische System zur Abbildung des Operationsfeldes vorgesehen. Über den Laserstrahlaufbau hinaus ist in das Behandlungsgerät 1 zusätzlich ein (in Fig. 1 der Einfachheit halber nicht dargestelltes) Beobachtungsmikroskop 20 integriert, das ein Objektfeld F des Auges 2 auf einen Empfänger 21 abbildet. Der Empfänger 21 kann dabei beispielsweise als CCD-Kamera realisiert werden. Anstelle des Empfängers 21 kann natürlich auch ein Okular eingesetzt werden, das auf eine Zwischenbildebene fokussiert ist. Zur Beleuchtung für das Beobachtungsmikroskop 20 ist der Adapter 12 so ausgebildet, daß er das Objektfeld F mit Beleuchtungsstrahlung beaufschlagt. Der Adapter 12 ist dazu zweiteilig aufgebaut. Er umfaßt ein Kontaktglas 22, das an der Augenhornhaut 17 anliegt und dieser die gewünschte Soll-Form gibt, sowie eine Fassung 23 für das Kontaktglas 22. Die Fassung 23 weist einen Koppler 24 auf, an dem ein (in Fig. 3 nicht näher bezeichneter) Lichtleiter angebracht ist, der Beleuchtungsstrahlung 25 in die Fassung 23 einbringt.

Die Fassung 23 leitet (in noch näher zu beschreibender Art und Weise) die zugeführte Beleuchtungsstrahlung 25 zum Objektfeld F, so daß dieses zur Beobachtung mit dem Beobachtungsmikroskop 20 ausreichend beleuchtet ist. In Fig. 3 ist zur Veranschaulichung schematisch ein Beleuchtungsstrahl 26 eingezeichnet, der auf die Iris 27 des Auges 2 fällt.

Das Beobachtungsmikroskop 20 befindet sich ebenso wie das Kontaktglas 22 auf der optischen Achse A1 des Behandlungsgerätes 1. Das Kontaktglas 22 dient damit nicht nur zur Führung der Beobachtungsstrahlung des Beobachtungsmikroskops 20, sondern auch zur Applizierung des Behandlungsstrahls 4, wie zuvor bereits allgemein für den Adapter 12 erläutert. Die Oberseite 31 des zentralen Bereichs bzw. des Kontaktglases 22 stellt dabei eine bekannte optische Grenzfläche für das Beobachtungsmikroskop 20 bereit.

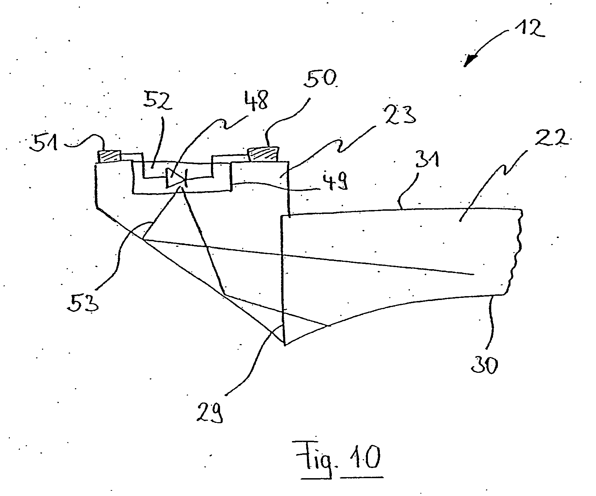

Fig. 4 zeigt den Adapter 12 schematisch in einer Schnittdarstellung. Wie zu sehen ist, hält die Fassung 23 das Kontaktglas 22 und weist an ihrer Unterseite einen Saugkanal 28 auf, der es erlaubt, die Fassung 23 mittels Unterdruck auf der Augenhornhaut 17 zu fixieren. Das Kontaktglas 22 ist dabei in die Fassung 23 eingeklebt, wobei ein optisch transparenter Kleber zur Anwendung kommt, dessen Brechzahl zwischen der Brechzahl des Materials des Kontaktglases 22 und der Brechzahl des Materials der Fassung 23 liegt. Damit treten beim Strahlungsübergang von der Fassung 23 in das Kontaktglas 22 nur minimale Reflexionsverluste auf.

Durch Ansaugen der Fassung 23 auf die Augenhornhaut 17 wird das in die Fassung 23 geklebte Kontaktglas 22 mit seiner Unterseite 30 auf die Augenhornhaut 17 gepreßt, so daß die zuvor bereits erläuterte gewünschte Soll-Form 19 gewährleistet ist.

Anstelle der zweiteiligen Ausführung gemäß Fig. 4 kann der Adapter 12 auch einstückig ausgebildet werden, wie Fig. 5 zeigt. Fassung 23 und Kontaktglas 22 sind dabei aus einem durchgehenden Teil hergestellt, beispielsweise durch Spritzgußverfahren oder durch ein zerspanendes Verfahren aus einem einzigen Rohteil. Die hier beschriebenen Varianten des Adapters 12 können grundsätzlich mehrteilig oder auch einteilig realisiert werden, insbesondere was Kontaktglas 22 und Fassung 23 angeht.

Fig. 6 zeigt vergrößert einen Ausschnitt des Kontaktglases 22 mit Fassung 23. Am Koppler 24 austretende Beleuchtungsstrahlen 26 werden über Reflexionen zur Unterseite 30 des Kontaktglases 22 geleitet und treten dort zur Beleuchtung des Objektfeldes aus. Dazu ist an der Fassung 23 ein reflektierender Rand 32 vorgesehen, der so ausgebildet ist, daß auf ihn auftreffende Beleuchtungsstrahlen 26 unterhalb des Brewsterwinkels auftreten und somit durch totale innere Reflexion reflektiert werden.

Zur Erzeugung der Beleuchtungsstrahlung ist eine Beleuchtungseinheit 33 vorgesehen, mit einem Glühemitter 34, oder Gasentladungseinheit, einer Luminiszenzdiode oder einer LED zur Abgabe der Beleuchtungsstrahlung 25. Die Beleuchtungsstrahlung 25 wird über eine Optik 35 in einen Lichtleiter 36 eingekoppelt, der einen Kern 37 mit relativ hoher Brechzahl und einen Mantel 38 mit relativ niedriger Brechzahl aufweist. Solche Lichtleiter sind im Stand der Technik bekannt. Sie führen die Strahlung im Lichtleiter durch totale innere Reflexion. Der Lichtleiter 36 ist an seinem Ende mit einer Hülse versehen, die in den Koppler 24 eingesteckt ist, so daß die Beleuchtungsstrahlung 25 in Form von Beleuchtungsstrahlung 26 vom Lichtleiter 36 in die Fassung 23 eingekoppelt wird. Die in Fig. 6 gezeigte Lage des Kopplers 24 ist beispielhaft. Der Koppler kann auch andernorts an der Fassung angeordnet werden, z.B. am Rand.

Die Beleuchtungsstrahlen 26 treffen nach einer oder mehrerer Reflexionen auf die Unterseite 30 des Kontaktglases 22. Zusätzlich oder alternativ zu dem unter geeignetem Winkel vorgesehenen Rand 32 können auch geeignete Verspiegelungen an der Fassung 23 vorgesehen werden, beispielsweise im Bereich des Saugkanals 28.

Das Austrittsverhalten der Beleuchtungsstrahlen 26 an der Unterseite 30 hängt ganz wesentlich davon ab, ob das Kontaktglas 22 am Auge anliegt oder nicht. Liegt das Kontaktglas 22 (noch) nicht am Auge an, ist die Differenz zwischen Brechzahlen des Kontaktglases 22 und der Umgebung (in der Regel Luft) sehr groß, und nur steil auf die Grenzfläche 20 treffende Strahlen können das Kontaktglas 22 verlassen. Liegt das Kontaktglas 22 dagegen am Auge an, ist die Brechzahldifferenz sehr gering und nur sehr flach auf die Grenzfläche 20 auftreffende Strahlen werden reflektiert. Die Einkopplung einer merklichen Strahlungsmenge in die Hornhaut 17 erfolgt also erst, wenn das Kontaktglas 22 auf die Hornhaut 17 gelegt wird.

Fig. 7 zeigt eine Schnittdarstellung ähnlich der Fig. 6, wobei die Beleuchtungseinheit 33 hier nicht mit eingezeichnet wurde. Wie bei der Ausführungsform gemäß Fig. 6 wird auch hier die Beleuchtungsstrahlung von der Fassung 23 über den Rand in das Kontaktglas 22 eingekoppelt. In Abwandlung zur Ausführungsform der Fig. 6 ist hier in der Fassung 23 ein Kanal 39 vorgesehen, der die Strahlung zum Kontaktglas 22 leitet.

Um die Beleuchtungsstrahlung 25 in den Kanal 39 einzukoppeln, ist für die am Lichtleiter 36 divergierend abgestrahlte Beleuchtungsstrahlung 25 eine Linse 40 mit konvexer Vorderfläche 41 und planer Rückfläche 42 vorgesehen. Nach der Linse 40 liegen somit im wesentlichen parallelisierte Beleuchtungsstrahlen 26 vor, die unter einem definierten Winkel auf eine im Kanal 39 vorgesehene Spiegelfläche 43 treffen. Dadurch ist erreicht, daß die Beleuchtungsstrahlen 26 mit nahezu gleichförmigem Winkel auf die Unterseite 30 des Kontaktglases 22 auftreffen.

Eine Kombination der den Bauweisen der Fig. 6 und 7 zugrundeliegenden Prinzipien zeigt Fig. 8. Hier ist zur Kollimierung der Beleuchtungsstrahlen eine abbildende Eintrittsfläche 44 an der Fassung 23 vorgesehen. Zwei (nicht näher bezeichnete) Spiegelflächen an der Fassung 23 sorgen dann wiederum für den gewünschten flachen Einfall auf die Unterseite 30 des Kontaktglases 22.

Die in der Fassung 23 vorgesehenen Spiegelflächen können je nach verwendetem Einfallswinkelbereich unterschiedlich ausgestaltet sein. Sind die Einfallswinkel auf diese Flächen groß, z.B. nahezu streifend, können die Flächen unbeschichtet bleiben und man kann die totale innere Reflexion ausnutzen. Sind die Einfallswinkel allerdings, wie in Fig. 8 eingezeichnet, eher so, daß ein Einfall nahe der Senkrechten erfolgt, kann die Reflektivität durch Aufbringen von metallischen oder dielektrischen Spiegelschichten gewährleistet werden. Eine weitere vorteilhafte Ausgestaltung erlaubt es, die in Fig. 8 lediglich als kegelige Spiegelflächen ausgebildeten Grenzflächen mit optischen Abbildungseigenschaften zu versehen. Auch kann es sich bei den Spiegelflächen um innerhalb der Fassung 23 gelegene Flächen handeln, sie müssen nicht zwingend, wie in Fig. 8 gezeichnet, an der Außenseite liegen.

Fig. 9 zeigt eine weitere alternative Ausgestaltung des Adapters, wobei hier eine Beleuchtung des Auges auch bereits vor dem Aufsetzen des Kontaktglases 22 erfolgt. Dazu treten die Beleuchtungsstrahlen 26 nach geeigneter Kollimierung durch eine abbildende Eintrittsfläche 44 an einer schräg zur Strahlausbreitungsrichtung liegenden Austrittsfläche 45 aus. Die Lage der Austrittsfläche 45 ist so gewählt, daß die Beleuchtungsstrahlen 26 als Strahlbündel 46 in Richtung auf eine zu beleuchtende Stelle B abgelenkt an der Fassung 23 austreten. Die Achse der durch die Austrittsfläche 45 definierten Ausbreitungsrichtung des Lichtbündels 46 stellt die optische Achse der Beleuchtung dar. Damit kann die Richtung der Beleuchtung so gewählt werden, daß sie auf eine vorbestimmte Stelle trifft, die es zu beleuchten gilt. In der Anwendung bei dem LASIK-Verfahren kann somit das Auge 2 beleuchtet werden, während das Kontaktglas aufgesetzt wird.

Die Austrittsfläche 45 kann auch auf beliebige vorbestimmte Weise gekrümmt gestaltet werden, um dem Bündel 42 eine gewünschte Divergenz zu verleihen. Damit kann man die Größe der beleuchteten Stelle B einstellen.

Um die Fassung 23 am Auge 2 zu befestigen, weist sie in der Ausführungsform der Fig. 9 an der Unterseite einen Kragen 47 auf, der gemeinsam mit der Austrittsfläche 45 den Saugkanal bildet.

Die unterschiedlichen Bauweisen, wie sie exemplarisch anhand der Fig. 6 bis 9 erläutert wurden, können an einer Fassung 23 natürlich mehrfach vorgesehen werden. Es ist also durchaus möglich und je nach Beleuchtungsaufgabe sogar auch besonders zweckmäßig, mehrere Einkoppelstellen, d.h. Koppler 24, vorzusehen, so daß an mehreren Stellen der Fassung 23 Beleuchtungsstrahlung eingekoppelt wird. Dabei müssen die verschiedenen Einkopplungen nicht alle dieselbe Ausführungsform, beispielsweise eine anhand der Fig. 6 bis 9 geschilderten realisieren, sondern es sind auch beliebige Kombinationen denkbar. Beispielsweise kann eine Bauweise zur Beleuchtung des Auges 2 bei noch nicht aufgesetztem Kontaktglas, wie sie in Fig. 9 exemplarisch beschrieben wurde, mit Bauweisen kombiniert werden, die erst bei aufgesetztem Kontaktglas 22 Beleuchtungsstrahlen in die Augenhornhaut 17 einkoppeln. Dies kann insbesondere vorteilhaft sein, falls das über die Austrittsfläche 45 in den Saugkanal eingekoppelte Bündel 46 nicht ausreicht, um bei aufgesetztem Kontaktglas 22 das Objektfeld zu beleuchten. Bei solchen Multibeleuchtungs-Adaptoren kann man während der Anwendung die Beleuchtung betriebsabhängig gestalten. Z. B. kann vor dem Aufsetzen die Beleuchtung gemäß dem Konzept der Fig. 9, nach dem Aufsetzen gemäß einem der anderen Konzepte erfolgen. Die Steuerung übernimmt zweckmäßigerweise das Steuergerät 14.

Alternativ zur Einkopplung der Beleuchtungsstrahlung 25 mittels eines oder mehrerer Lichtleiter 36 kann auch eine Fassung 23 verwendet werden, die selbst über Mittel zur Erzeugung der Beleuchtungsstrahlung verfügt. Ein solches Beispiel ist in Fig. 10 dargestellt. Die Fassung 23 ist mit einer LED 48 ausgestaltet, welche sich in einer Vertiefung 49 der Fassung 23 befindet. Die LED 48 ist über Kontakte 50 und 51 kontaktiert.

Die LED 48 ist in die Vertiefung 49 mit einem transparenten, flüssig verarbeitbaren aushärtenden Material 52, z.B. einem Epoxid-Harz, eingebetet. Die Kontaktstellen können als kleine Kontaktpads ausgeführt werden oder sich um den ganzen Umfang der Fassung 23 erstrecken, um eine Kontaktierung in beliebiger Rotationsstellung zu ermöglichen. Bei Kontaktpads können, um eine Kontaktierung in beliebiger Rotationsstellung zu gewährleisten, alternativ auch ringförmige Gegenkontakte eingesetzt werden.

Der Weg der von der LED 48 ausgehenden Strahlung 53 folgt den anhand der Fig. 6 bis 9 dargestellten Prinzipien, d.h. die Strahlung 53 wird direkt aus der Fassung 23 auf das Auge 2 gestrahlt, oder über das Kontaktglas 22 an der Unterseite 30 abgestrahlt. Natürlich sind auch hier Kombinationen möglich.

## Patentansprüche

1. Adapter (12) zum Koppeln einer Laserbearbeitungsvorrichtung (1) mit einem Objekt (17), der aufweist:
- einen zentralen Bereich (22), der In den Strahlengang der Laserbearbeitungsvorrichtung (1) schaltbar ist, wobei der zentrale Bereich ein Kontaktglas (22) zum Aufliegen auf das Objekt (17) aufweist,
- einen Beleuchtungsstrahlengang, durch den Beleuchtungsstrahlung (25) zur Beleuchtung eines durch den zentralen Bereich (22) erfaßbaren Objektfeldes leitbar ist, und
- einen außerhalb des zentralen Bereiches (22) liegenden Randbereich, mit dem der Adapter (12) am Objekt (17) und/oder an der Laserbearbeitungsvorrichtung (1) befestigbar ist, wobei der Randbereich eine Fassung (23) des Kontaktglases (22) umfaßt,
**dadurch gekennzeichnet, daß** die Fassung (23) ein für Beleuchtungsstrahlung (25) transparentes Material, insbesondere PMMA, Polycarbonat, Zeonex oder HW 55, aufweist und daß mindestens eine am Randbereich vorgesehene Einkoppeleinrichtung (24) für Beleuchtungsstrahlung (25) vorgesehen ist, wobei der Beleuchtungsstrahlengang im Randbereich verläuft und in den Randbereich eingekoppelte Beleuchtungsstrahlung (25) direkt und/oder über den zentralen Bereich (22) zum Objektfeld leitet .

2. Adapter nach Anspruch 1, **dadurch gekennzeichnet, daß** die Einkoppeleinrichtung (24) abbildende Wirkung für die Beleuchtungsstrahlung (25) hat, insbesondere eine konvexe, konkave, zylindrische und/oder torische Grenzfläche (44) aufweist.

3. Adapter nach Anspruch 1 oder 2, **dadurch gekennzeichnet, daß** die Einkoppeleinrichtung (24) zum Zwecke der spektralen Filterung oder Reflexreduzierung eine dielektrische Schicht aufweist.

4. Adapter nach einem der obigen Ansprüche, **dadurch gekennzeichnet, daß** der Randbereich (23) eine die Beleuchtungsstrahlung (25) reflektierende Fläche, insbesondere Außenfläche, aufweist.

5. Adapter nach den Ansprüchen 2 und 4, **dadurch gekennzeichnet, daß** mehrere Einkoppeleinrichtungen (24) vorgesehen sind, die reflektierende Fläche in Facetten segmentiert ist und jeder Einkoppeleinrichtung eine Facette oder eine Gruppe von Facetten zugeordnet ist.

6. Adapter nach Anspruch 4 oder 5, **dadurch gekennzeichnet, daß** die reflektierende Fläche abbildende Wirkung für die Beleuchtungsstrahlung (25) hat.

7. Adapter nach Anspruch 1, **dadurch gekennzeichnet, daß** zwischen Fassung (23) und Kontaktglas (22) eine Klebeschicht vorgesehen ist, insbesondere eine Klebeschicht mit einer Brechzahl, die zwischen der Brechzahl der Fassung (23) und der des Kontaktglases (22) liegt.

8. Adapter nach Anspruch 1, **dadurch gekennzeichnet, daß** die Einkoppeleinrichtung eine am Randbereich (23) befestigte oder in den Randbereich (23) eingelassene LED (28) umfaßt.

9. Adapter nach Anspruch 8, **dadurch gekennzeichnet, daß** am Randbereich (23) Kontakte (50, 51), insbesondere ring förmige Kontakte, vorgesehen sind, die eine Kontaktierung in beliebiger Drehstellung zur Laserbearbeitungsvorrichtung (1) ermöglichen.

10. Adapter nach einem der obigen Ansprüche, **dadurch gekennzeichnet, daß** zentraler Bereich (22) und Randbereich (23) in einem einstückigen Bauteil realisiert sind.

## Claims

1. An adapter (12) for coupling a laser processing device (1) to an object (17), said adapter comprising:
- a central region (22) which can be moved into the beam path of the laser processing device (1), wherein the central region comprises a contact glass (22) to be placed on the object (17),
- an illumination beam path through which illumination radiation (25) can be guided for illumination of an object field which can be sensed through the central region (22), and
- a peripheral region which is located outside the central region (22) and by which the adapter (12) can be mounted to the object (17) and/or to the laser processing device (1), wherein the peripheral region comprises a mount (23) of the contact glass (22),
**characterized in that** the mount (23) comprises a material which is transparent for illumination radiation (25), in particular PMMA, polycarbonate, Zeonex or HW 55, and that at least one coupling unit (24) for illumination radiation (25) is provided at the peripheral region, wherein the illumination beam path extends in the peripheral region and carries illumination radiation (25), coupled into the peripheral region, to the object field directly and/or via the central region (22).

2. The adapter according to claim 1, **characterized in that** the coupling unit (24) has an imaging effect for the illumination radiation (25) and comprises, in particular, a convex, concave, cylindrical and/or toroidal interface (44).

3. The adapter according to claim 1 or 2, **characterized in that** the coupling unit (24) comprises a dielectric layer for the purpose of spectral filtering or reduction of reflections.

4. The adapter according to any one of the above claims, **characterized in that** the peripheral region (23) comprises a surface which reflects the illumination radiation (25), in particular an outside surface.

5. The adapter according to any one of claims 2 and 4, **characterized in that** a plurality of coupling units (24) are provided, the reflecting surface is segmented into facets and each coupling unit has a facet or group of facets assigned to it.

6. The adapter according to claim 4 or 5, **characterized in that** the reflecting surface has an imaging effect for the illumination radiation (25).

7. The adapter according to claim 1, **characterized in that** an adhesive layer is provided between the mount (23) and the contact glass (22), in particular an adhesive layer having a refractive index which is between the refractive index of the mount (23) and that of the contact glass (22).

8. The adapter according to claim 1, **characterized in that** the coupling unit comprises an LED (28) mounted to or embedded in the peripheral region (23).

9. The adapter according to claim 8, **characterized in that** contacts (50, 51), in particular annular contacts, are provided at the peripheral region (23), which contacts allow contact to be made in any rotary position relative to the laser processing device (1).

10. The adapter according to any one of the above Claims, **characterized in that** the central region (22) and the peripheral region (23) are realized in one integral component.

## Revendications

1. Adaptateur (12) pour coupler un dispositif d'usinage par laser (1) à un objet (17), lequel comporte :
- une zone centrale (22), qui peut être montée dans la trajectoire de rayonnement du dispositif d'usinage par laser (1), la zone centrale comportant un verre de contact (22) destiné à être posé sur l'objet (17),
- une trajectoire du faisceau d'éclairage, par laquelle le faisceau d'éclairage (25) peut être guidé pour éclairer un champ de l'objet, à saisir par la zone centrale (22), et
- une zone de bordure, qui est située en dehors de la zone centrale (22) et par laquelle l'adaptateur (12) peut être fixé sur l'objet (17) et/ou sur le dispositif d'usinage par laser (1), la zone de bordure comportant une douille (23) du verre de contact (22),
**caractérisé en ce que** la douille (23) comporte un matériau transparent pour le faisceau d'éclairage (25), en particulier le PMMA, le polycarbonate, le zeonex ou le HW 55, et **en ce qu'**il est prévu dans la zone de bordure au moins un dispositif d'injection (24) pour le faisceau d'éclairage (25), la trajectoire du faisceau d'éclairage s'étendant dans la zone de bordure et guide le faisceau d'éclairage (25), injecté dans la zone de bordure, directement et/ou par l'intermédiaire de la zone centrale (22) vers le champ de l'objet.

2. Adaptateur selon la revendication 1, **caractérisé en ce que** le dispositif d'injection (24) exerce une action de projection pour le faisceau d'éclairage (25), en particulier comporte une interface (44) convexe, concave, cylindrique et/ou torique.

3. Adaptateur selon la revendication 1 ou 2, **caractérisé en ce que** le dispositif d'injection (24) comporte une couche diélectrique à des fins de filtrage spectral ou de diminution de la réflexion.

4. Adaptateur selon l'une quelconque des revendications précédentes, **caractérisé en ce que** la zone de bordure (23) comporte une surface réfléchissant le faisceau d'éclairage (25), en particulier une surface extérieure.

5. Adaptateur selon les revendications 2 et 4, **caractérisé en ce qu'**il est prévu plusieurs dispositifs d'injection (24), la surface réfléchissante est segmentée en facettes et une facette ou un groupe de facettes est associé à chaque dispositif d'injection.

6. Adaptateur selon la revendication 4 ou 5, **caractérisé en ce que** la surface réfléchissante exerce une action de projection pour le faisceau d'éclairage (25).

7. Adaptateur selon la revendication 1, **caractérisé en ce qu'**il est prévu entre la douille (23) et le verre de contact (22) une couche de colle, en particulier une couche de colle avec un indice de réfraction qui est situé entre l'indice de réfraction de la douille (23) et celui du verre de contact (22).

8. Adaptateur selon la revendication 1, **caractérisé en ce que** le dispositif d'injection comporte une diode électroluminescente (28) fixée sur la zone de bordure (23) ou insérée dans la zone de bordure (23).

9. Adaptateur selon la revendication 8, **caractérisé en ce que** dans la zone de bordure (23) sont prévus des contacts (50, 51), en particulier des contacts annulaires, qui permettent d'établir le contact dans n'importe quelle position de rotation par rapport au dispositif d'usinage par laser (1).

10. Adaptateur selon l'une quelconque des revendications précédentes, **caractérisé en ce que** la zone centrale (22) et la zone de bordure (23) sont réalisées sous forme de pièce d'un seul tenant.
